Europäisches Patentamt

European Patent Office

Office européen des brevets

(19)

(11) Veröffentlichungsnummer : **0 472 869 B1**

(12) **EUROPÄISCHE PATENTSCHRIFT**

(45) Veröffentlichungstag der Patentschrift :
10.11.93 Patentblatt 93/45

(51) Int. Cl.⁵ : **C12N 15/77**

(21) Anmeldenummer : **91111532.7**

(22) Anmeldetag : **11.07.91**

(54) Neue Plasmide aus Corynebacterium glutamicum und davon abgeleitete Plasmidvektoren.

(30) Priorität : **30.08.90 DE 4027453**

(43) Veröffentlichungstag der Anmeldung :
**04.03.92 Patentblatt 92/10**

(45) Bekanntmachung des Hinweises auf die
Patenterteilung :
**10.11.93 Patentblatt 93/45**

(84) Benannte Vertragsstaaten :
**BE DE DK FR GB IT NL SE**

(56) Entgegenhaltungen :
DE-A- 3 402 876
BIOTECHNOLOGY, Band 5, Februar 1987, Seiten 137-146, NY, US; J.F. MARTIN et al.: "Cloning systems in amino acid-producingcorynebacteria"
J. BACTERIOLOGY, Band 162, Nr. 2, Mai 1985, Seiten 591-597, Baltimore, US; M. YOSHIHAMA et al.: "Cloning vector system for corynebacterium glutamicum"
GENE, Band 107, Nr. 1, 30. Oktober 1991, Seiten 69-74, Amsterdam, NL; H. SONNEN et al.: "Characterization of pGA1, a new plasmid from corynebacterium glutamicum LP-6"

(73) Patentinhaber : **DEGUSSA AG**
**Weissfrauenstrasse 9**
**D-60311 Frankfurt (DE)**

(72) Erfinder : **Thierbach, Georg, Dr.**
**Gunststrasse 21**
**W-4800 Bielefeld 1 (DE)**
Erfinder : **Kautz, Petra-Sabine**
**Grafenheiderstrasse 54**
**W-4800 Bielefeld (DE)**
Erfinder : **Kutzner, Hans Jürgen, Prof. Dr.**
**Dresdner Strasse 16**
**W-6001 Oberramstadt (DE)**
Erfinder : **Sonnen, Hans**
**Modaustrasse 33**
**W-6086 Riedstadt 3 (DE)**
Erfinder : **Bachmann, Frank**
**Platanenallee 46**
**W-6082 Mörfelden-Waldorf (DE)**
Erfinder : **Pühler, Alfred, Prof. Dr.**
**Am Waldschlösschen 2**
**W-4800 Bielefeld (DE)**
Erfinder : **Schäfer, Andreas**
**Bünderstrasse 33**
**W-4800 Bielefeld (DE)**

## Beschreibung

Die Erfindung betrifft neue Plasmide aus Corynebacterium glutamicum, die kompatibel sind und davon abgeleitete Plasmidvektoren (Pendelvektoren).

Für die gentechnische Stammverbesserung sind Plasmidvektoren essentielle Voraussetzung. Die Konstruktion von Plasmidvektoren für Corynebacterium bzw. Brevibacterium beruht im allgemeinen auf kryptischen Plasmiden, die in dieser Gruppe von Bakterien gefunden werden können.

Plasmidvektoren für Corynebacterium und Brevibacterium können dazu benutzt werden, Gene der Biosynthese von Aminosäuren zu klonieren, das entsprechende Genprodukt bzw. Enzym in einem gesteigerten Maß zu exprimieren und dadurch die Aminosäuren-Ausscheidung zu verbessern. Beispielhaft sei genannt die Verbesserung der Ausscheidung von L-Lysin durch Corynebacterium glutamicum durch Klonierung und Überexpression des Phosphoenolpyruvat-Carboxylase-Gens von Corynebacterium glutamicum (Europäische Patentanmeldung No. 89 114 632.6).

Plasmide sind in der Gruppe der coryneformen Aminosäuren-ausscheidenden Bakterien nur sehr selten zu finden, auch wenn bei Durchsicht der Literatur ein gegenteiliger Eindruck entstehen könnte.

Bei genauerem Hinsehen zeigt sich nämlich, daß unter unterschiedlichen Namen beschriebene Plasmide so viele gleiche Eigenschaften aufweisen, daß sie als identisch anzusehen sind.

Als Beispiele seien die Plasmide pAM286 aus C.glutamicum AJ11560 (EP-A-0 77 548); pAM330 aus B.lactofermentum ATCC13869 (EP-A-0 77 548), pBL1 aus B.lactofermentum ATCC21798 (Santamaria, R. et al., J. Gen. Microbiol. 130, 2237-2246 (1984))und pX18 aus B.lactofermentum ATCC21086 (Yeh, P. et al., Gene 47, 301-308(1986))genannt.

Gleiches gilt für die Plasmide pHM1519 aus C.glutamicum ATCC13058 (EP-A-0 78 537), pCG1 aus C.glutamicum ATCC31808 (EP-A-0 58 889), pRN 3.1 aus C.glutamicum ATCC39269 (DE-A-3402876) und pSR1 (Yoshihama, M. et al., J.Bact. 162, 591-597 (1985)) aus C.glutamicum ATCC 19223. Auch hier zeigt die hohe Übereinstimmung der zu den jeweiligen Plasmiden veröffentlichten Daten dem Fachmann, daß es sich um die gleichen Plasmid-Spezies handeln muß. Martin, J. F. et al., (Bio/Technology 5, 137-146 (1987))bestätigen diese Annahme.

Die Zahl der Veröffentlichungen und Patentanmeldungen zu diesem Thema zeigt aber, welches Interesse an einer Vielfalt von Plasmiden besteht, die zur Entwicklung von Klonierungssystemen für coryneforme Bakterien geeignet sind.

Von besonderer Bedeutung für die Entwicklung z. B. Aminosäuren-ausscheidender Stämme sind Plasmide, die nebeneinander in einer Zelle existieren können.

Derartige kompatible Plasmide erlauben es, Kombinationen biosynthetischer Gene gleichzeitig in einem Mikroorganismus einzubringen, die gewünschten Enzymaktivitäten anzuheben und so die Bildung des erwünschten Produkts wie z. B. L-Threonin oder L-Lysin zu verbessern. Besonders günstige Voraussetzungen dafür ergäben sich beispielsweise bei kompatiblen Plasmiden, von denen das eine eine hohe und das andere eine niedrige Kopienzahl aufweist, um eine ausgewogene Überexpression der jeweiligen Gene zu erreichen und eine unnötige Belastung des Wirts zu vermeiden.

Gleichzeitig muß natürlich auch eine ausreichende Stabilität der verwendeten Plasmide angestrebt werden.

Wie aus der Literatur bekannt ist, existieren aber keine Kompatibilitätsstudien (Martin, J.F. et al., loc. cit., 139), ebensowenig wie Untersuchungen zur Stabilität von Plasmidvektoren.

Aufgabe der Erfindung ist es, Plasmide bzw. Plasmidvektoren bereitzustellen, die kompatibel sind und ausreichende Stabilität aufweisen, um die methodischen Voraussetzungen zur gentechnischen Stammverbesserung insbesondere von coryneformen Aminosäure -ausscheidenden Bakterien über den Stand der Technik hinaus zu verbessern.

Gegenstand der Erfindung ist das Plasmid pGA1, isoliert aus Corynebacterium glutamicum LP-6, hinterlegt unter der Nummer DSM 5816 , charakterisiert durch eine Länge von~ 4,9 Kb und folgende Restriktionsschnittstellen:

## Tabelle 1

| Restriktionsenzyme | Anzahl der Schnittstellen | DNA-Fragmente (Kb) | | |
|---|---|---|---|---|
| Apa I | 0 | – | | |
| Bam H I | 2 | 3,3 | 1,6 | |
| Bcl I | 0 | – | | |
| Bgl I | 0 | – | | |
| Bgl II | 0 | – | | |
| Bst E II | 1 | 4,9 | | |
| Cla I | 1 | 4,9 | | |
| Dra I | 0 | – | | |
| Eco R I | 1 | 4,9 | | |
| Eco R V | 0 | – | | |
| Hind III | 4 | 2,4  1,1  1,05  0,3 | | |
| Kpn I | 0 | – | | |
| Mlu I | 3 | 2,45 | 2,25 | 0,2 |
| Pvu II | 2 | 3,25 | 1,65 | |
| Sal I | 0 | – | | |
| Sph I | 1 | 4,9 | | |
| Sst I | 0 | – | | |
| Xba I | 2 | 2,5 | 2,4 | |
| Xho I | 0 | – | | |

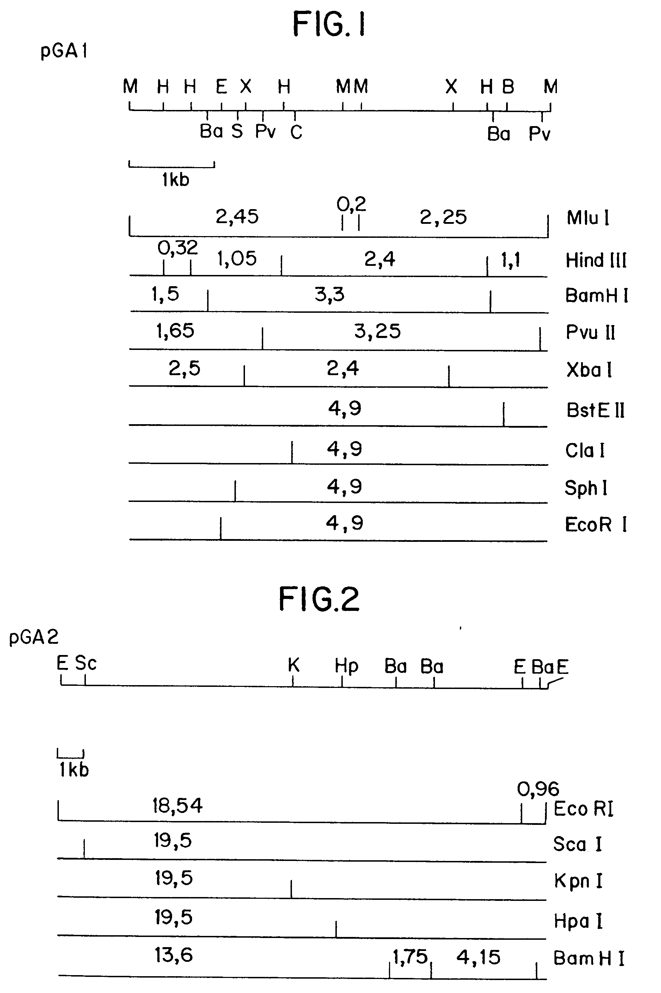

Fig. 1 enthält die Restriktionskarte von pGA1 in linearer Darstellung.

Corynebacterium glutamicum Stamm LP-6 wurde vom "Felix d'Herelle Reference Center for Bacterial Viruses" in Quebec 10,Quebec Laval University, Canada GIK 7P4 unter der Nummer HER1229 bezogen. C-.glutamicum Stamm LP-6 wurde bei der Deutschen Sammlung von Mikroorganismen in Braunschweig, Bundesrepublik Deutschland, gemäß dem Budapester Vertrag als DSM 5816 hinterlegt.

Ein weiterer Gegenstand ist das mit pGA1 kompatible Plasmid pGA2, isoliert aus Corynebacterium glutamicum LP-6, hinterlegt unter der Nummer DSM 5816 charakterisiert durch eine Länge von~ 19,5 Kb und folgende Restriktionsschnittstellen:

## Tabelle 2

| Restriktionsenzyme | Anzahl der Schnittstellen | DNA-Fragmente (Kb) | | | | |
|---|---|---|---|---|---|---|
| Bam H I | 3 | 13,6 | 4,15 | 1,75 | | |
| Eco R I | 2 | 18,5 | 0,96 | | | |
| Hind III | 5 | 6,61 | 5,72 | 3,31 | 2,04 | 1,82 |
| Hpa I | 1 | 19,5 | | | | |
| Kpn I | 1 | 19,5 | | | | |
| Mlu I | 3 | 15,8 | 2,84 | 0,82 | | |
| Sca I | 1 | 19,5 | | | | |
| Sma I | 4 | 14,5 | 3,34 | 1,00 | 0,65 | |
| Xho I | 0 | – | | | | |

Fig. 2 enthält die Restriktionskarte von pGA2 in linearer Darstellung.

Die Plasmid-DNA kann aus dem hinterlegten Stamm nach dem Fachmann bekannten Methoden isoliert werden.

Während das Plasmid pGA1 eine hohe Kopienzahl (~50 pro Zelle) aufweist, wird für pGA2 eine niedrige Kopienzahl (~5) bestimmt.

Als Wirt für diese Plasmide dienen coryneforme Bakterien, insbesondere Aminosäuren produzierende Bakterien.

Beispiele der coryneformen Bakterien sind:

```
Brevibacterium flavum, insbesonders          ATCC 14067
Brevibacterium lactofermentum, insbesonders  ATCC 13869
Corynebacterium callunae, insbesonders       ATCC 15991
Corynebacterium glutamicum, insbesonders     ATCC 13032
Corynebacterium melassecola, insbesonders    ATCC 17965
Corynebacterium thermoaminogenes, insbesonders  Ferm P-9244
```

Da die erfindungsgemäßen Plasmide sich in den Zellen der coryneformen Bakterien vermehren, sind sie in der Lage, die Information von fremden in die Plasmide inserierten Genen in den Wirtsstellen zu amplifizieren.

Der Einbau der rekombinanten Plasmid-DNA in die Wirtszellen erfolgt bevorzugt über zusammengesetzte Plasmide (Plasmidvektoren), zu deren Konstruktion besonders geeignet sind ein Resistenzgen aufweisende, in E-coli replizierende Vektoren, wie z. B. pACYC177, pACYC184, pSC101, pBR322, pIP55, R16, R1, RP4 und pIE545.

Das Prinzip derartiger Konstruktionen wird in der EP-A-93611 und EP-A-82 485 beschrieben.

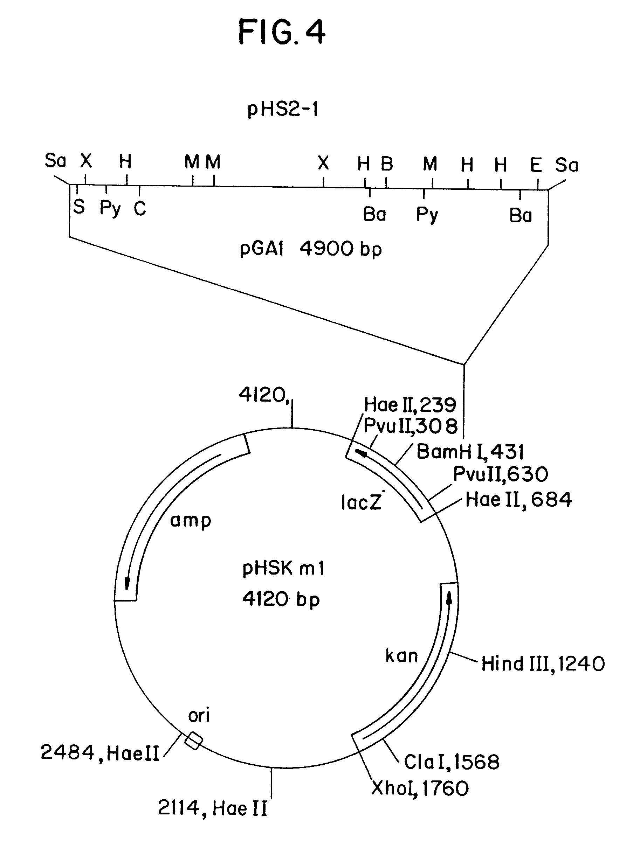

Besonders geeignet ist der Pendelvektor pHS 2-1, bestehend aus dem Plasmid pGA1 und dem E.coli-Vektor pHSKml, charakterisiert durch die Restriktionskarte entsprechend Fig. 4.

Der Vektor pHSKm1 ist ein Derivat des E.coli-Vektors pUC18, das bei Yanish-Perron, C. et al., (Gene 33, 103-119 (1985)) beschrieben ist, in welchen das Kanamycin-Resistenzgen des Transposon Tn 903 inseriert wurde.

Dieser und andere auf pGA1 basierende Pendelvektoren sind nicht nur stabil in coryneformen Bakterien, insbesondere Corynebacterium glutamicum, sondern koexistieren auch mit Plasmiden anderen Ursprungs, wie z. B. pHM1519 aus Corynebacterium glutamicum ATCC 13058 und pAM 330 aus Brevibacterium lactofermentum ATCC 13869, insbesondere mit pCC1 aus Corynebacterium callunae DSM 20147.

Dies ist auch deshalb überraschend, weil es sich in den genannten Fällen um ein Plasmid bzw. einen Vektor mit hoher Kopienzahl handelt.

Der Nachweis erfolgte auf diesem Weg:

Brevibacterium lactofermentum ATCC 13869, der das Plasmid pAM 330 trägt, wurde wie bei Thierbach et al., (Appl. Microbiol. Biotechnol. 29, 356-362 (1988)) beschrieben, mit pHS2-1-DNA transformiert.

Die Untersuchung der Transformanten ergab, daß beide Plasmide koexistieren können.

Ein mobibilisierbares Derivat des Plasmids pGA1 wurde mit Hilfe der Konjugationsmethode wie bei Schäfer et al., (J. Bact. 172, 1663-1666 (1990)) beschrieben, in Corynebacterium glutamicum ATCC13058, der das Plasmid pHM1519 trägt, eingeführt. Die Untersuchung der Transkonjuganten ergab, daß beide Plasmide koexistieren können.

Auch das Plasmid pGA2 ist zur konstruktion von mobilisierbaren Pendelvektoren geeignet. Aus einer DNA-Sequenz von pGA2 und dem mobilisierbaren E.coli-Vektor pK18:: mob wird der Pendelvektor pFBH2 konstruiert, der in Corynebacterium glutamicum repliziert.

### 1. Isolierung und Charakterisierung von pGA1 aus Corynebacterium glutamicum LP-6

Corynebacterium glutamicum Stamm LP-6 wurde vom "Felix d'Hérelle Reference Center for Bacterial Viruses" in Quebec 3 10, Quebec Laval University, Canada G1K 7P4 unter der Nummer HER1229 bezogen. C. glutamicum Stamm LP-6 wurde bei der Deutschen Sammlung von Mikroorganismen in Braunschweig, Bundesrepublik Deutschland, gemäß dem Budapester Vertrag als DSM5816 hinterlegt.

Plasmid-DNA wurde aus Stamm LP-6 nach dem Fachmann bekannten Methoden, wie sie zum Beispiel in der Europäischen Patentanmeldung 0 318 663 und bei Birnboim, H.C. und Doly, J. (Nucleic Acids Research 7, 1513-1523 (1979)) beschrieben sind, isoliert. Die auf diese Weise erhaltene DNA-Lösung wurde durch Agarosegel-Elektrophorese (Maniatis, T. et al. (1982), Molecular Cloning: A Laboratory Manual, Cold Spring Harbor) charakterisiert. Die DNA-Lösung enthielt zwei Plasmid-Spezies, die als pGA1 und als pGA2 bezeichnet wurden. Plasmid pGA1 hat eine Länge von ungefähr 4,9 Kb und Plasmid pGA2 von ungefähr 19,5 Kb. Durch Behandlung mit dem Restriktionsenzym SalI wurde das Plasmid pGA2 gespalten, während pGA1 intakt blieb. Die dadurch erhaltene DNA-Lösung wurde einer CsCl/Ethidiumbromid-Dichtegradientenzentrifugation (Maniatis, T. et al. (1982), Molecular Cloning: A Laboratory Manual, Gold Spring Harbor) unterworfen und das Plasmid pGA1 auf diese Weise isoliert.

Plasmid pGA1 wurde mit den Restriktonsenzymen Apal, BamHI, Bcll, Bgll, BglIl, BstEll, Clal, Dral, EcoRl, EcoRV, HindIll, Kpnl, Mlul, Pvull, Sall, Sphl, Sstl, Xbal und Xhol behandelt und die Länge der DNA-Fragmente durch Agarosegel-Elektrophorese und Längenvergleich mit dem Fachmann bekannten Standard DNA-Fragmenten bestimmt. Ein solcher Standard ist zum Beispiel DNA des Escherichia coli Phagen λ, die mit dem Restriktionsenzym HindIll gespalten worden ist und von der Firma Bethesda Research Laboratories in Gaithersburg, USA bezogen werden kann. Tabelle 1 gibt die Anzahl der Restriktonsschnittstellen in Plasmid pGA1 fur die untersuchten Restriktionsenzyme und die Länge der erhaltenen DNA-Fragmente an. Durch Verdauung von pGA1 mit zwei und drei Restriktionsenzymen und Längenbestimmung der erhaltenen DNA-Fragmente konnte eine Restriktionskarte des Plasmids aufgestellt werden, die in Abbildung 1 dargestellt ist. Plasmid pGA1 hat eine Länge von 4,9 Kb.

Die Kopienzahl von pGA1 in Stamm LP-6 wurde folgendermaßen bestimmt: Stamm LP-6 wurde in StandardI-Bouillon (bezogen von der Firma Merck, Darmstadt, FRG) bis zum Beginn der stationären Wachstumsphase kultiviert und die Zellzahl bestimmt. Aus 1,5 ml-Kultursuspension wurde die Plasmid-DNA, wie bei Birnboim, H.C. und Doly, J. (Nuclear Acids Research 7, 1513-1523 (1979)) beschrieben, isoliert. Ein Aliquot der Plasmid-DNA-Lösung und eine bekannte Menge des oben beschriebenen λ-HindIll-DNA-Standards wurden einer Agarosegel-Elektrophorese mit anschließender Ethidiumbromid-Färbung unterworfen. Die Fluoreszenz bei UV-Bestrahlung der pGA1-DNABande wurde mit der der λ-HindIll-DNA-Fragmente verglichen und so die Menge an pGA1-DNA halbquantitativ bestimmt. Auf diese Weise wurde die Kopienzahl (Anzahl der Plasmidmoleküle pro Zelle) als ca. 50 bestimmt.

### 2. Isolierung und Charakterisierung von pGA2 aus Corynebacterium glutamicum LP-6

Eine Plasmid-DNA-haltige Lösung mit den Plasmiden pGA1 und pGA2 wurde, wie unter 1. beschrieben, hergestellt. Die beiden Plasmid-Spezies wurden durch Agarosegel-Elektrophorese aufgetrennt und Plasmid pGA2 durch Elektroelution (Maniatis, T. et al, (1982), Molecular Cloning: A Laboratory Manual, Cold Spring Harbor) isoliert.

Plasmid pGA2 wurde mit den Restriktionsenzymen EcoRl, BamHI, Kpnl, Scal, Hpal, Mlul, Smal, HindIll und Xhol behandelt und die Länge der DNA-Fragmente, wie unter 1. beschrieben, bestimmt. Tabelle 2 gibt die Anzahl der Restriktonsschnittstellen in Plasmid pGA2 fur die untersuchten Restriktionsenzyme und die Länge der erhaltenen DNA-Fragmente an. Durch Verdauung von pGA2 mit zwei und drei Restriktionsenzymen und Längenbestimmung der erhaltenen DNA-Fragmente konnte eine Restriktionskarte des Plasmids aufgestellt werden, die in Abbildung 2 dargestellt ist. Plasmid pGA2 hat eine Länge von 19,5 kb.

Die Kopienzahl von pGA2 in Stamm LP-6 wurde folgendermaßen bestimmt: Stamm LP-6 wurde in StandardI-Bouillon (bezogen von der Firma Merck, Darmstadt, FRG) bis zum Beginn der stationären Wachstumsphase kultiviert und die Zellzahl bestimmt. Aus 1,5 ml-Kultursuspension wurde die Plasmid-DNA, wie bei Birnboim, H.C. und Doly, J. (Nucleic Acids Research 7, 1513-1523 (1979)) beschrieben, isoliert. Ein Aliquot der Plasmid-DNA-Losung und eine bekannte Menge des oben beschriebenen λ-HindIll-DNA-Standards wurden einer Agarosegel-Elektrophorese mit anschließender Ethidiumbromid-Färbung unterworfen. Die Fluoreszenz bei UV-Bestrahlung der pGA2-DNA-Bande wurde mit der der λ-HindIll-DNA-Fragmente verglichen und so die Menge an pGA2-DNA halbquantitativ bestimmt. Auf diese Weise wurde die Kopienzahl (Anzahl der Plasmidmoleküle pro Zelle) als ca. 5 bestimmt.

| Restriktions-enzyme | Anzahl der Schnitt-stellen | DNA-Fragmente (kb) | | | | |
|---|---|---|---|---|---|---|
| BamHI | 3 | 13,6 | 4,15 | 1,75 | | |
| EcoRI | 2 | 18,5 | 0,96 | | | |
| HindIII | 5 | 6,61 | 5,72 | 3,31 | 2,04 | 1,82 |
| HpaI | 1 | 19,5 | | | | |
| KpnI | 1 | 19,5 | | | | |
| MluI | 3 | 15,8 | 2,84 | 0,82 | | |
| ScaI | 1 | 19,5 | | | | |
| SmaI | 4 | 14,5 | 3,34 | 1,00 | 0,65 | |
| XhoI | 0 | - | | | | |

Tab. 2: Charakterisierung des Plasmids pGA2 mittels Restriktionsendonukleasen.

### 3. Konstruktion des Pendelvektors pHS2-1 bestehend aus pGA1 und dem Escherichia coli-Vektor pHSKm1

Plasmid pHSKm1 ist ein Derivat des dem Fachmann bekannten E. coli-Vektors pUC18, das bei Yanisch-Perron, C. et al. (Gene 33, 103-119 (1985)) beschrieben und bei der Firma Pharmacia in Uppsala, Schweden erhältlich ist, in welches das Kanamycin-Resistenzgen des Transposon Tn903 (Oka, A. et al, J. Mol. Biol. 147, 217-226 (1981)) inseriert wurde.

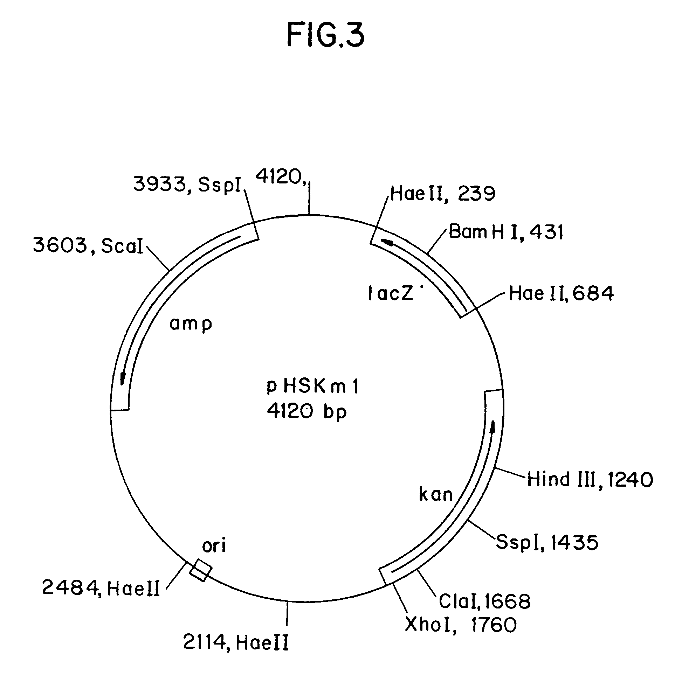

Plasmid pHSKm1 wurde, wie im folgenden beschrieben, konstruiert. Plasmid pAGYC177, welches das Kanamycin-Resistenzgen des Transposon Tn903 trägt (Rose, R.E.; Nucleic Acids Research 16, 356 (1988)), wurde aus Escherichia coli ATCC37031, wie in EPA 0 316 663 beschrieben, isoliert. Plasmid pACYC177 wurde mit dem Restriktionsenzym HaeII gespalten und das 1430 bp lange DNA-Fragment, welches das Kanamycin-Resistenzgen trägt, durch Elektroelution (Maniatis, T. et al, (1982), Molecular Cloning: A Laboratory Manual. Cold Spring Harbor) isoliert. Plasmid pUC18 wurde partiell mit HaeII gespalten, mit dem 1430 bp langen HaeII-DNA-Fragment gemischt und das resultierende DNA-Gemisch mit T4-DNA-Ligase behandelt. Kompetente Zellen von Escherichia coli DH5α, die von der Firma Bethesda Research Laboratories in Gaithersburg, USA bezogen wurden, wurden mit dem Ligationsgemisch transformiert. Kanamycin-resistente Transformanten wurden auf LB-Agar (Trypton: 10 g/l; Hefeextrakt: 5 g/l; Nacl: 10 g/l; Agar: 12 g/l) der mit 20 µg/ml Kanamycin supplementiert worden war, selektioniert. Aus einer Transformante wurde das als pHSKm1 bezeichnete Plasmid isoliert. Durch Restriktionsanalyse wurde die Position und Orientierung des inserierten 1430 bp langen DNA-Fragmentes mit dem Kanamycin-Resistenzgen bestimmt. Die Restriktionskarte des Plasmids pHSKm1 ist in Abbildung 3 dargestellt.

Plasmid pGA1, das unter 1. beschrieben ist, wurde mit dem Restriktionsenzym Sau3A partiell verdaut und die linearisierte und einmal geschnittene Form des Plasmids durch Elektroelution isoliert. Die auf diese Weise erhaltene DNA wurde mit pHSKm1-DNA, die mit BamHI linearisiert worden war, gemischt und das resultierende DNA-Gemisch mit T4-DNA-Ligase behandelt. Escherichia coli DH5α wurde mit dem Ligationsgemisch transformiert und Transformanten auf LB-Agar selektioniert, der mit Kanamycin (20 µg/ml), X-Gal (5-Brom-4-chlor-3-indolyl-D-galactopyranosid, 40 µg/ml und IPTG (Isopropyl-β-D-thiogalactopyranosid, 20 µg/ml) supplementiert worden war. Ungefähr 5 % der Transformanten waren auf dem beschriebenen Agar farblos. Eine Koloniehybridisierung mit Plasmid pGA1 als Sonde wurde mit ungefähr 900 Transformanten durchgefuhrt, die keine lacZα-Komplementation zeigen. Für die Koloniehybridisierung wurde pGA1 mittels "nick"-Translation (Maniatis, T. et al. (1982), Molecular Cloning: A Laboratory Manual, Cold Spring Harbor) mit biotinylierten Nucleosidtriphosphaten (Biotin-7-dATP) markiert und mit Hilfe des BluGENE Nukleinsäure-Nachweissystems der Firma Bethesda Research Laboratories in Gaithersburg, USA durch die Streptavidin gekoppelte alkalische Phosphatase nachgewiesen. Die Markierung der DNA wurde wie bei Langer et al. (Proceedings of the National Academy of Sciences, USA, 80, 6633-6637 (1981)) und die Koloniehybridisierung wie bei Trevor, G.T. (Microbiological Methods 3, 259 pp. (1985)) beschrieben, durchgeführt. Von den 900 geprüften Transformanten gaben 70 eine positive Reaktion. Aus einer Transformante wurde das als pHS2-1 bezeichnete Plasmid isoliert und mit Hilfe von Restriktionsenzymen kartiert. Die Restriktionskarte von Plasmid pHS2-1 ist in Abbildung 4 dargestellt.

### 4. Replikation des Pendelvektors pHS2-1 in Corynebacterium glutamicum ATCC13032

Plasmid pHS2-1 wurde aus Escherichia coli DH5α/pHS2-1 isoliert und Sphäroplasten von Corynebacterium glutamicum ATCC13032, wie bei Thierbach et al. (Appl. Microbiol. Biotechnol. 29, 356-362 (1988)) beschrieben, damit transformiert. Transformanten wurden auf SB-Agar selektioniert, der Kanamycin (15 µg/ml) enthielt. Plasmid-DNA wurde aus 12 Transformanten isoliert und durch Spaltung mit den Restriktionsenzymen EcoRI und MluI charakterisiert. Die Plasmid-DNA hatte die gleiche Größe und zeigte das gleiche Restriktionsmuster wie pHS2-1, isoliert aus Escherichia coli.

### 5. Stabilität des Pendelvektors pHS2-1 in Corynebacterium glutamicum ATCC13032

Corynebacterium glutamicum ATCC13032/pHS2-1 wurde in StandardI-Nährbouillon (bezogen von der Firma Merck, Darmstadt, BRD), die mit 4 g/l Glucose und zusätzlich mit 10 µg/ml Kanamycin supplementiert worden war, bei 30°C und 150 rpm bis zum Erreichen der stationären Wachstumsphase kultiviert. Eine 50 ml-Kultur bestehend aus oben beschriebenem Nährmedium mit Kanamycin und eine 50 ml-Kultur bestehend aus dem oben beschriebenen Nährmedium ohne Kanamycin wurden jeweils im Verhältnis 1:10.000 mit der Vorkultur beimpft und, wie oben beschrieben, bis zum Erreichen der stationären Wachstumsphase kultiviert. Die optische Dichte der Bakterien-Kultur betrug 7 bis 8 bei einer Meßwellenlänge von 660 nm.

Anschließend wurde die Kanamycin-haltige Bakterienkultur im Verhältnis 1:10.000 auf frische Kanamycinhaltige Nährbouillon überimpft und die Bakterienkultur, die in Abwesenheit von Kanamycin gewachsen war, im gleichen Verhältnis auf frische Kanamycin-freie Nährbouillon überimpft. Insgesamt wurden 16 derartige Überimpfungen durchgeführt, so daß der Stamm für ca. 210 Generationen in An- bzw. in Abwesenheit von Kanamycin kultiviert wurde. Danach wurden Proben auf StandardI-Agar (bezogen von der Firma Merck, Darmstadt, BRD) ausplattiert und bei 30°C bebrütet. Die sich bildenden Einzelkolonien wurden auf StandardI-Agar und auf StandardI-Agar, der mit 10 µg/ml Kanamycin supplementiert worden war, überstempelt. Im Falle der Kultur für 210 Generationen in Abwesenheit von Kanamycin waren 181 von 181 untersuchten Einzelkolonien Kanamycin-resistent, und im Falle der Kultur in Gegenwart von Kanamycin waren 84 von 84 untersuchten Einzelkolonien Kanamycin-resistent. Plasmid-DNA wurde aus jeweils 12 Einzelkolonien isoliert und durch Spaltung mit den Restriktionsenzymen EcoRI und MluI charakterisiert. Die Plasmid-DNA hatte die Große von pHS2-1 und zeigte das fur pHS2-1 charakteristische Restriktionsmuster.

### 6. Koexistenz des Plasmids pCC1 aus Corynebacterium callunae DSM20147 mit dem Pendelvektor pHS2-1

Corynebacterium callunae DSM20147 wurde mit pHS2-1 Plasmid-DNA, isoliert aus Corynebacterium glutamicum ATCC13032/pHS2-1, wie bei Thierbach et al. (Appl. Microbiol. Biotechnol. 29, 356-362 (1988)) beschrieben, transformiert. Transformanten wurden auf SB-Agar selektioniert, der Kanamycin (15 µg/ml) enthielt. 20 Transformanten wurden auf StandardI-Nähragar, der mit Kanamycin (10 µg/ml) supplementiert worden war, vereinzelt. Jeweils eine Einzelkolonie wurde abgenommen und Plasmid-DNA isoliert. In 17 von 20 untersuchten Einzelkolonien vom Typ DSM20147/pHS2-1 wurde das Plasmid pCC1 durch Agarosegel-Elektrophorese nachgewiesen; Plasmid pHS2-1 war in allen untersuchten Klonen vorhanden.

In gleicher Weise wurden als Kontrolle Sphäroplasten von Corynebacterium callunae DSM20147 hergestellt und auf SB-Agar regeneriert. 20 regenerierte Einzelkolonien wurden auf StandardI-Nähragar vereinzelt. Jeweils eine Einzelkolonie wurde abgenommen und Plasmid-DNA isoliert. In 17 von 20 untersuchten Einzelkolonien vom Typ DSM20147 wurde das Plasmid pCC1 nachgewiesen. Eine Transformante vom Typ DSM20147/pHS2-1 wurde für ungefähr 50 Generationen in An- bzw. Abwesenheit von Kanamycin -so, wie unter 5. beschrieben - kultiviert. Anschließend wurden die beiden Kulturen auf StandardI-Agar ausplattiert und bei 30°C bebrütet. Plasmid-DNA wurde aus jeweils 8 Einzelkolonien isoliert. In jeweils 8 von 8 untersuchten Kolonien konnten die Plasmide pCC1 und pHS2-1 nachgewiesen werden. In gleicher Weise wurde die Kontrolle eine regenerierte Einzelkolonie von Stamm DSM20147 für ungefähr 50 Generationen kultiviert und anschließend an die Plasmid-DNA aus 8 Einzelkolonien isoliert. In jeweils 8 von 8 untersuchten Einzelkolonien wurde das Plasmid pCC1 nachgewiesen. Die Untersuchung zeigte, daß die Plasmide pCC1 und pHS2-1 koexistieren können.

### 7. Ausscheidung von L-Lysin durch Corynebacterium glutamicum Stamm LP-6

Stamm LP-6 wurde in einem Medium kultiviert, das aus 12 g/l Ammoniumsulfat, 240 g/l Melasse und 60 ml/l Sojabohnenmehlhydrolysat bestand und dessen pH auf ca. 7,2 mit ammoniakalischem Wasser eingestellt worden war. 100 ml-Erlenmeyerkolben wurden mit 10 ml des oben beschriebenen Mediums befüllt, mit Stamm LP-6 beimpft und 72 Stunden bei 30°C und 300 rpm inkubiert. Die Bestimmung von L-Lysin erfolgte im zentrifugierten Überstand mit Hilfe von Aminosäure-Analysatoren. Die ausgeschiedene Konzentration an L-Lysin·HCl war 0,9 g/l.

### 8. Konstruktion des mobilisierbaren Pendelvektors pFBH2 bestehend aus einer DNA-Sequenz aus pGA2 und dem mobilisierbaren Escherichia coli-Vektor pK18::mob

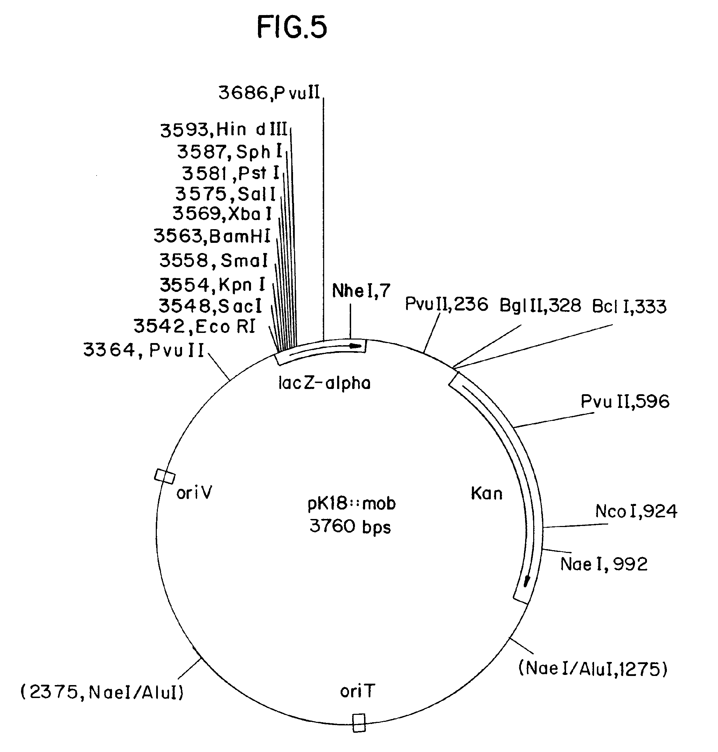

Plasmid pK18::mob ist ein Derivat des dem Fachmann bekannten Escherichia coli Kloniervektors pK18 (Pridmore, R.D., Gene 56, 309-312 (1987)). Plasmid pK18::mob trägt die für die Mobilisierung essentielle Region des selbsttransferierbaren Plasmids RP4 (Datta, N. et al., J. Bact. 108, 1244-1249 (1971)) und wurde wie folgt konstruiert: Plasmid pSUP102, welches die Mobilisierungsregion des Plasmides RP4 trägt (Simon, R. et al., Methods in Enzymology 118, 640-659 (1986)), wurde aus Escherichia coli S17-1 (Simon, R. et al., Biotechnology 1, 784-794 (1983)) nach dem Fachmann geläufigen Verfahren, wie z.B. bei Holmes, D.S. & Quigley, M. (Analyt. Biochem. 114, 193-197 (1981)) beschrieben, isoliert und mit dem Restriktionsenzym AluI partiell gespalten. Plasmid pK18 wurde mit dem Restriktionsenzym NaeI partiell verdaut, mit den durch AluI-Verdauung des Plasmids pSUP102 erhaltenen DNA-Fragmenten vereinigt und das resultierende DNA-Gemisch mit T4-DNA-Ligase behandelt. Kompetente Zellen von Escherichia coli S17-1, die nach der Vorschrift von Cohen, S, et al. (Proc. Natl. Acad. Sci. USA 69, 2110-2114 (1972)) hergestellt worden waren, wurden mit dem Ligationsgemisch transformiert und die Zellen auf PA-Agar, bestehend aus 17,5 g/l Penassay-Broth (Difco Laboratories, Detroit, USA) und 15 g/l Agar aufgebracht, dem Kanamycin mit einer Endkonzentration von 25 µg/ml hinzugefügt worden war. Kanamycin-resistente Kolonien wurden mit PA-Flüssigmedium abgeschwemmt und in einer größeren Menge PA-Flüssigmedium angezogen und nach gängigen Methoden, wie bei Simon, R. et al. (Biotechnology 1, 784-794 (1983)) beschrieben, zur Kreuzung mit Escherichia coli MM294 (Talmadge, K. and Gilbert, W., Gene 12, 235-241 (1980)) eingesetzt. Aus den auf Selektionsmedium (PA-Agar mit 25 µg/ml Kanamycin und 50 µg/ml Nalidixinsäure) gewachsenen Transkonjuganten konnten Derivate des Plasmids pK18 isoliert werden, die Insertionen von pSUP102-DNA trugen. Der hier als pK18::mob bezeichnete Vektor trägt eine pSUP102-Insertion von 1,1 kb Länge. Durch Restriktionsanalyse wurde die Position des inserierten DNA-Fragmentes mit der Mobilisierungsregion ermittelt. Die Restriktionskarte des Plasmids pK18::mob ist in Abbildung 5 dargestellt.

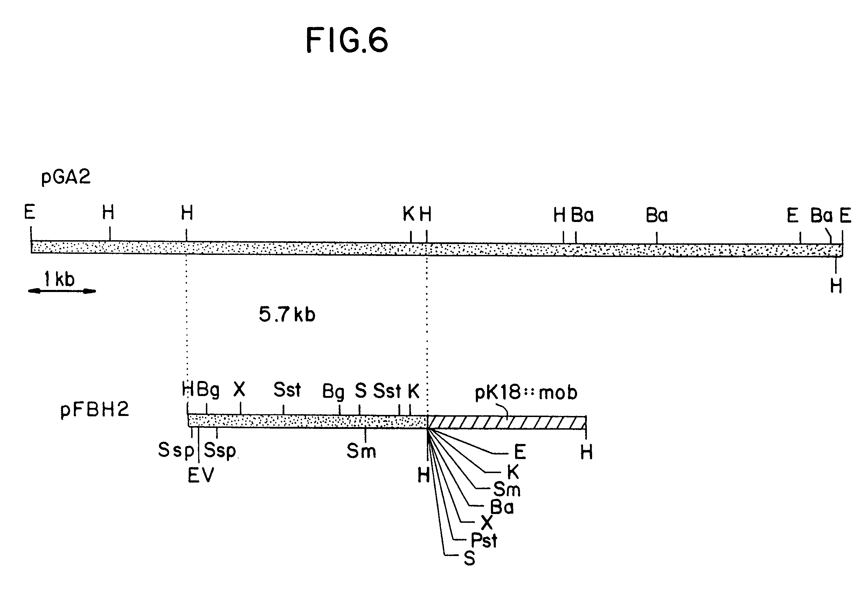

Plasmid pGA2, das unter 2. beschrieben ist, wurde mit dem Restriktionsenzym HindIII gespalten und die resultierenden pGA2 DNA-Fragmente mit dem Plasmidvektor pK18::mob, welcher vorher mit HindIII linearisiert worden war, gemischt. Das resultierende DNA-Gemisch wurde mit T4-DNA-Ligase behandelt und anschließend zur Transformation von Escherichia coli DH5α eingesetzt. Transformanten wurden auf LB-Agar selektioniert, der mit Kanamycin (20 µg/ml), X-Gal (40 µg/ml) und IPTG (20 µg/ml) supplementiert worden war. Alle farblosen Kolonien wurden mittels dem Fachmann bekannter Plasmid-Präparationen, wie sie zum Beispiel bei Birnboim, H.C. und Doly, J. (Nucleic Acids Research 7, 1513-1523, (1979)) beschrieben sind, auf Plasmidgehalt überprüft und die Plasmide mit Hilfe von Restriktionsenzymen charakterisiert. Eines der isolierten Plasmide trug eine pGA2-Insertion von 5,7 kb Länge und wurde als pFBH2 bezeichnet. Die Restriktionskarte von pFBH2 ist in Abbildung 6 dargestellt.

### 9. Replikation des Pendelvektors pFBH2 in Corynebacterium glutamicum RM3

Escherichia coli S17-1 wurde mit pFBH2-DNA - isoliert aus DH5α/pF8H2 - transformiert, und die resultierenden Transformanten auf LB-Agar mit Kanamycin (20 µg/ml) selektioniert. Eine Transformante vom Typ S17-1/pFBH2 wurde zur Konjugation mit Corynebacterium glutamicum RM3, wie bei Schäfer et al. (J. Bact. 172, 1663-1666, (1990)) beschrieben, eingesetzt. Transkonjuganten von C. glutamicum RM3 wurden auf LB-Agar selektioniert, der mit Kanamycin (20 µg/ml) und Nalidixinsäure (50 µg/ml) supplementiert worden war. Plasmid-DNA wurde aus 12 Transkonjuganten isoliert und durch Spaltung mit verschiedenen Restriktionsenzymen charakterisiert. Die isolierte Plasmid-DNA hatte die gleiche Größe und zeigte das gleiche Restriktionsmuster wie pFBH2, isoliert aus Escherichia coli.

Beschreibung der Figuren

Fig. 1: Restriktionskarte des Plasmids pGA1 in linearer Darstellung.
Abkürzung: Ba, BamHI; B, BstEII; C, ClaI; E, EcoRI; H, HindIII; M, MluI; Pv, PvuII; S, SphI; X, XbaI.

Fig. 2: Restriktionskarte des Plasmids pGA2 in linearer Darstellung.
Abkürzungen: Ba, BamHI; E, EcoRI; Hp, HpaI; K, KpnI; Sc, ScaI.

Fig. 3: Restriktionskarte des Plasmids pHSKml in zirkulärer Darstellung.
Die multiple Klonierstelle erstreckt sich zwischen den Positionen 400 bis 452, Abkürzungen: amp, Ampicillin-Resistenzgen; Kan, Kanamycin-Resistenzgen; lacZ', 5'-terminale Teil des lacZ-Gens, der lacZα-Komplementation erlaubt; ori, Replikationsursprung.

Fig. 4: Restriktionskarte des Plasmids pHS2-1 in linearer Darstellung. Der pGA1- und der pHSKml-Teil 1 sind getrennt dargestellt. Der pGA1-Teil von pHS2-1 enthält mindestens 6 HaeII-Restriktionsschnittstellen, die nicht eingezeichnet wurden.
Abkürzungen: Sa, Sau3A; alle übrigen Abkürzungen sind in Fig. 1 erklärt.

Fig. 5: Restriktionskarte des Plasmids pK18::mob in zirkulärer Darstellung. Abkürzungen: Kan, Kanamycin-Resistenzgen; lacZ-alpha, 5'-terminaler Teil des lacZ-Gens, der lacZα-Komplementation erlaubt; oriV, Replikationsursprung; oriT, Ursprung für den konjugativen Plasmidtransfer (Mobilisierungsregion). Der DNA-Bereich zwischen den Positionen (2375, NaeI/AluI) und (1275, NaeI/AluI) trägt 3 NaeI-Restriktionsschnittstellen, die nicht kartiert wurden.

Fig. 6: Restriktionskarte des Plasmids pFBH2 in linearer Darstellung. Abkürzungen: Ba, BamHI; Bg, BglII; E, EcoRI; EV, EcoRV; H, HindIII; K, KpnI; Pst, PstI; Sm, SmaI; S, SphI; Ssp, SspI; Sst, SstI; X, XbaI. Der pGA2-Teil des Moleküls ist durch graue Schattierung und der pK18::mob-Teil durch Schraffur gekennzeichnet.

**Patentansprüche**

1. Miteinander kompatible Plasmide pGA1 und pGA2, isoliert aus Corynebacterium glutamicum LP-6, hinterlegt unter der Nummer DSM 5616, wobei das Plasmid pGA1 charakterisiert ist durch eine Länge von ~ 4.9 Kb und folgende Restriktionsschnittstellen:

| Restriktionsenzyme | Anzahl der Schnittstellen | DNA-Fragmente (Kb) | | |
|---|---|---|---|---|
| Apa I | 0 | – | | |
| Bam H I | 2 | 3,3 | 1,6 | |
| Bcl I | 0 | – | | |
| Bgl I | 0 | – | | |
| Bgl II | 0 | – | | |
| Bst E II | 1 | 4,9 | | |
| Cla I | 1 | 4,9 | | |
| Dra I | 0 | – | | |
| Eco R I | 1 | 4,9 | | |
| Eco R V | 0 | – | | |
| Hind III | 4 | 2,4  0,3 | 1,1 | 1,05 |
| Kpn I | 0 | – | | |
| Mlu I | 3 | 2,45 | 2,25 | 0,2 |
| Pvu II | 2 | 3,25 | 1,65 | |
| Sal I | 0 | – | | |
| Sph I | 1 | 4,9 | | |
| Sst I | 0 | – | | |
| Xba I | 2 | 2,5 | 2,4 | |
| Xho I | 0 | – | | |

und das Plasmid pGA2 charakterisiert ist durch eine Länge von ~ 19.5 Kb und folgende Restriktionsschnittstellen:

| Restriktions enzyme | Anzahl der Schnitt- stellen | DNA-Fragment (Kb) | | | | |
|---|---|---|---|---|---|---|
| BamH I | 3 | 13,6 | 4,15 | 1,75 | | |
| EcoR I | 2 | 18,5 | 0,96 | | | |
| Hind III | 5 | 6,61 | 5,72 | 3,31 | 2,04 | 1,82 |
| Hpa I | 1 | 19,5 | | | | |
| Kpn I | 1 | 19,5 | | | | |
| Mlu I< | 3 | 15,8 | 2,84 | 0,82 | | |
| Sca I | 1 | 19,5 | | | | |
| Sma I | 4 | 14,5 | 3,34 | 1,00 | 0,65 | |
| Xho I | 0 | – | | | | |

2. Zur autonomen Replikation in coryneformen Bakterien befähigtes rekombinantes Plasmid. konstruiert aus Plasmid pGA1 nach Anspruch 1 durch Hinzufügen mindestens eines Fremd-DNA-Fragments.

3. Zur autonomen Replikation in coryneformen Bakterien befähigtes rekombinantes Plasmid., konstruiert aus Plasmid pGA2 nach Anspruch 2 durch Hinzufügen mindestens eines Fremd-DNA-Fragments.

4. Rekombinantes Plasmid (Plasmidvektor) gemäß den Ansprüchen 2 oder 3, zusammengesetzt aus
a) dem Plasmid pGA1 oder pGA2 oder einer aus diesen Plasmiden geschnittenen DNA-Sequenz.
b) einem ein Resistenzgen tragenden DNA-Fragment. das aus einem in E.coli replizierenden Vektor. geschnitten wurde.

5. Plasmidvektor pHS2-1 gemäß Anspruch 4, bestehend aus dem Plasmid pGA1 und dem E.coli-Vektor pHSKm1, charakterisiert durch die Restriktionskarte entsprechend Fig. 4.

6. Plasmidvektor pFBH2 gemäß Anspruch 4, bestehend aus einer DNA-Frequenz aus pGA2 und dem mobilisierbaren E.coli-Vektor pK18::mob, charakterisiert durch die Restriktionskarte entsprechend Fig. 5 und Fig. 6.

7. Rekombinantes Plasmid gemäß einem der Ansprüche 2 bis 6, welches zusätzlich ein in coryneformen Bakterien exprimierbares Gen enthält.

8. Coryneforme Bakterien, welche ein rekombinantes Plasmid gemäß einem der Ansprüche 2 bis 7 enthalten.

## Claims

1. Mutually compatible plasmids pGA1 and pGA2, isolated from Corynebacterium glutamicum LP-6 deposited under the number DSM 5616, plasmid pGA1 being characterized by a length of -4.9 kb and the following restriction cleavage sites:

| Restriction enzyme | Number of cleavage sites | DNA fragments (kb) | | |
|---|---|---|---|---|
| ApaI | 0 | – | | |
| BamHI | 2 | 3.3 | 1.6 | |
| BclI | 0 | – | | |
| BglI | 0 | – | | |
| BglII | 0 | – | | |
| BstEII | 1 | 4.9 | | |
| ClaI | 1 | 4.9 | | |
| DraI | 0 | – | | |
| EcoRI | 1 | 4.9 | | |
| EcoRV | 0 | – | | |
| HindIII | 4 | 2.4 | 1.1 | 1.05 |
| | | 0.3 | | |
| KpnI | 0 | – | | |
| MluI | 3 | 2.45 | 2.25 | 0.2 |
| PvuII | 2 | 3.25 | 1.65 | |
| SalI | 0 | – | | |
| SphI | 1 | 4.9 | | |
| SstI | 0 | – | | |
| XbaI | 2 | 2.5 | 2.4 | |
| XhoI | 0 | – | | |

and plasmid pGA2 being characterized by a length of ∼19.5 kb and the following restriction cleavage sites:

| Restriction enzyme | Number of cleavage sites | DNA fragments (kb) | | |
|---|---|---|---|---|
| BamHI | 3 | 13.6 | 4.15 | 1.75 |
| EcoRI | 2 | 18.5 | 0.96 | |
| HindIII | 5 | 6.61 | 5.72 | 3.31 |
| | | 2.04 | 1.82 | |
| HpaI | 1 | 19.5 | | |
| KpnI | 1 | 19.5 | | |
| MluI | 3 | 15.8 | 2.84 | 0.82 |
| ScaI | 1 | 19.5 | | |
| SmaI | 4 | 14.5 | 3.34 | 1.00 |
| | | 0.65 | | |
| XhoI | 0 | — | | |

2. A recombinant plasmid capable of autonomous replication in corynebacteria, constructed from plasmid pGA1 according to Claim 1 by the addition of at least one foreign DNA fragment.

3. A recombinant plasmid capable of autonomous replication in corynebacteria, constructed from plasmid pGA2 according to Claim 2 by the addition of at least one foreign DNA fragment.

4. A recombinant plasmid (plasmid vector) according to Claims 2 or 3, composed of
   a) plasmid pGA1 or pGA2 or a DNA sequence cleaved from these plasmids, and
   b) a DNA fragment carrying a resistance gene, which has been cleaved from a vector replicating in E. coli.

5. Plasmid vector pHS2-1 according to Claim 4, consisting of plasmid pGA1 and E. coli vector pHSKm1, characterized by the restriction map according to Fig. 4.

6. Plasmid vector pFBH2 according to Claim 4, consisting of a DNA sequence from pGA2 and mobilizable E. coli vector pK18::mob, characterized by the restriction map according to Fig. 5 and Fig. 6.

7. A recombinant plasmid according to one of Claims 2 to 6 which additionally contains a gene expressible in corynebacteria.

8. Corynebacteria which contain a recombinant plasmid according to one of Claims 2 to 7.

**Revendications**

1. Plasmides pGA1 et pGA2 compatibles l'un avec l'autre, isolés à partir de corynebacterium glutamicum LP-6 déposé sous le n° DSM 5616, le plasmide pGA1 étant caractérisé par une longueur de ~ 4,9 kb et par les sites de coupure suivants :

| Enzymes de restriction | Nombre des sites de coupure | Fragments d'ADN (kb) | | |
|---|---|---|---|---|
| Apa I | 0 | – | | |
| Bam H I | 2 | 3,3 | 1,6 | |
| Bcl I | 0 | – | | |
| Bgl I | 0 | – | | |
| Bgl II | 0 | – | | |
| Bst E II | 1 | 4,9 | | |
| Cla I | 1 | 4,9 | | |
| Dra I | 0 | – | | |
| Eco R I | 1 | 4,9 | | |
| Eco R V | 0 | – | | |
| Hind III | 4 | 2,4 | 1,1 | 1,05 |
| | | 0,3 | | |
| Kpn I | 0 | – | | |
| Mlu I | 3 | 2,45 | 2,25 | 0,2 |
| Pvu II | 2 | 3,25 | 1,65 | |
| Sal I | 0 | – | | |
| Sph I | 1 | 4,9 | | |
| Sst I | 0 | – | | |
| Xba I | 2 | 2,5 | 2,4 | |
| Xho I | 0 | – | | |

et le plasmide pGA2 étant caractérisé par une longueur de ~ 19,5 kb et par les sites de coupure suivants :

| Enzymes de restriction | Nombre des sites de coupure | Fragments d'ADN (kb) | | | | |
|---|---|---|---|---|---|---|
| Bam H I | 3 | 13,6 | 4,15 | 1,75 | | |
| Eco R I | 2 | 18,5 | 0,96 | | | |
| Hind III | 5 | 6,61 | 5,72 | 3,31 | 2,04 | 1,82 |
| Hpa I | 1 | 19,5 | | | | |
| Kpn I | 1 | 19,5 | | | | |
| Mlu I | 3 | 15,8 | 2,84 | 0,82 | | |
| Sca I | 1 | 19,5 | | | | |
| Sma I | 4 | 14,5 | 3,34 | 1,00 | 0,65 | |
| Xho I | 0 | – | | | | |

2.  Plasmide recombinant capable de réplication autonome dans des bactéries corynéformes, construit à partir du plasmide pGA1 selon la revendication 1, par addition d'au moins un fragment d'ADN étranger.

3.  Plasmide recombinant capable de réplication autonome dans des bactéries corynéformes, construit à partir du plasmide pGA2 selon la revendication 2, par addition d'au moins un fragment d'ADN étranger.

4.  Plasmide recombinant (vecteur plasmidique) selon la revendication 2 ou 3, composé
    a) du plasmide pGA1 ou pGA2 ou d'une séquence d'ADN coupée à partir de ces plasmides,
    b) d'un fragment d'ADN portant un gène de résistance, qui a été coupé à partir d'un vecteur se répliquant dans E. Coli.

5.  Vecteur plasmidique pHS2-1 selon la revendication 4, constitué du plasmide pGA1 et du vecteur-pHSKm1 de E. Coli, caractérisé par la carte de restriction correspondant à la figure 4.

6.  Vecteur plasmidique pFBH2 selon la revendication 4, constitué d'une fréquence d'ADN provenant de pGA2 et du vecteur mobilisable pK18 :: mob de E. Coli, caractérisé par la carte de restriction correspondant à la figure 5 et à la figure 6.

7.  Plasmide recombinant selon l'une quelconque des revendications 2 à 6, qui contient en outre un gène exprimable dans des bactéries corynéformes.

8.  Bactéries corynéformes contenant un plasmide recombinant selon l'une quelconque des revendications 2 à 7.

# FIG. I

pGA1

# FIG.2

pGA2

# FIG.3

# FIG. 4

pHS2-1

pGA1 4900 bp

pHSK m1
4120 bp

# FIG.5

3686,PvuII

3593,Hin dIII
3587,Sph I
3581,Pst I
3575,Sal I
3569,Xba I
3563,BamHI
3558,Sma I
3554,Kpn I
3548,Sac I
3542,Eco RI
3364,Pvu II

NheI,7

PvuII,236  BglII,328  Bcl I,333

lacZ-alpha

Pvu II,596

oriV

Kan

pK18::mob
3760 bps

Nco I,924

Nae I,992

(2375,NaeI/AluI)

oriT

(Nae I/AluI,1275)

# FIG.6